# EUROPEAN PATENT SPECIFICATION

(11) **EP 1 932 913 B1**
(45) Date of publication and mention of the grant of the patent: **16.01.2013**
(21) Application number: 07023905.8
(22) Date of filing: 11.12.2007
(51) Int. Cl.: C12N 15/10, C12Q 1/68

(54) **Nucleic acid isolation using polidocanol and derivatives**
Nukleinsäureisolation mithilfe von Polidocanol und Derivaten davon
Isolation d'acide nucléique utilisant du polidocanol et des dérivés

(30) Priority: 11.12.2006 EP 06025539
(43) Date of publication of application: 18.06.2008
(73) Proprietor: Roche Diagnostics GmbH, 68305 Mannheim (DE); F.Hoffmann-La Roche AG, 4070 Basel (CH)
(72) Inventor: Adie, Sigrid, 94099 Ruhstorf (DE); Leying, Hermann, 83673 Bichl (DE); Nachbaur, Nicole, 82418 Murnau (DE); Russmann, Eberhard, 82377 Penzberg (DE)
(74) Representative: Herren, Barbara

(56) References cited:
- WO-A-00/09746
- WO-A-01/37291
- WO-A-96/41811
- WO-A-2005/064010
- WO-A1-2005/012523
- WO-A1-2005/034967

## Description

### Field of the Invention

This invention relates to a composition comprising a chaotropic agent, a buffering substance, a protease, and 0.5 to 4.9 % (V/V) polidocanol or a derivative thereof. The invention is further related to uses of this composition and to a kit comprising the composition according to the invention. The invention is further related to a method for the detection of a nucleic acid in a biological sample comprising the steps of incubating the biological sample in the presence of a chaotropic agent, a buffering substance, a proteases, and 0.5 to 4.9 % (V/V) polidocanol or a derivative thereof, optionally isolating the nucleic acid, optionally amplifying the nucleic acid, and detecting the nucleic acid. The invention is further related to a method for the purification of a nucleic acid in a biological sample comprising the steps of incubating the biological sample in the presence of a chaotropic agent, a buffering substance, a protease, and 0.5 to 4.9 % (V/V) polidocanol or a derivative thereof and isolating the nucleic acid thereby purifying the nucleic acid.

### Background of the Invention

Many biological substances, especially nucleic acids, present special challenges in terms of isolating them from their natural environment. On the one hand, they are often present in very small concentrations and, on the other hand, they are often found in the presence of many other solid and dissolved substances e.g. after lysis of cells. This makes them difficult to isolate or to measure, in particular in biospecific assays which allow the detection of specific analytes, e.g. nucleic acids, or specific analyse properties and play a major role in the field of diagnostics and bioanalytics in research and development. Examples for biospecific assays are hybridisation assays, immuno assays and receptor-ligand assays. Hybridisation assays use the specific base-pairing for the molecular detection of nucleic acid analytes e.g. RNA and DNA. Hence, oligonucleotide probes with a length of 18 to 20 nucleotides may enable the specific recognition of a selected complementary sequence e.g. in the human genome. Another assay which entails the selective binding of two oligonucleotide primers is the polymerase chain reaction (PCR) described in US 4,683,195. This method allows the selective amplification of a specific nucleic acid region to detectable levels by a thermostable polymerase in the presence of desoxynucleotide triphosphates in several cycles.

As described above, before the biological substances may be analysed in one of the above-mentioned assays or used for other processes, it has to be isolated or purified from biological samples containing complex mixtures of different components as e.g. proteinaceous and non-proteinaceous components. Often, for the first steps, processes are used which allow the enrichment of the component of interest, e.g. the nucleic acid. Frequently, these are contained in a bacterial cell, a fungal cell, a viral particle, or the cell of a more complex organism, such as a human blood cell or a plant cell. The component of interest can also be called a "target component".

To release the contents of said cells or particles, they may be treated with enzymes or with chemicals to dissolve, degrade or denature the cellular walls of such organisms. This process is commonly referred to as lysis. The resulting solution containing such lysed material is referred to as lysate. A problem often encountered during the lysis is that other enzymes degrading the target component, e.g. desoxyribonucleases or ribonucleases degrading nucleic adds, come into contact with the component of interest during lysis. These degrading enzymes may also be present outside of the cells or may have been spatially separated in different cellular compartiments before the lysis and come now into contact with the target component. Other components released during this process may be e.g. endotoxins belonging to the family of lipopolysaccharides which are toxic to cells and can cause problems for products intended to be used in human or animal therapy.

There are a variety of means to tackle this problem mentioned-above. It is common to use chaotropic agents as e.g. guanidinium thiocyanate or anionic, cationic, zwitterionic or non-ionic detergents when nucleic acids are intended to be set free. It is also an advantage to use proteases which rapidly degrade these enzymes or unwanted proteins. However, this may produce another problem as the said substances or enzymes can interfere with reagents or components in subsequent steps.

Enzymes which can be advantageously used in such lysis or sample preparation processes mentioned-above are enzymes which cleave the amide linkages in protein substrates and which are classified as proteases, or (interchangeably) peptidases (See Walsh, C., Enzymatic Reaction Mechanisms (1979) chapter 3, W. H. Freeman and Company, San Francisco). Proteases which have been used are e.g. alkaline proteases (WO 98/04730) or acid proteases (US 5,386,024). The protease which is widely used for sample preparation for the isolation of nucleic acids is proteinase K from Tritirachium album (see e.g. Sambrook, J., et al.: Molecular Cloning (1989) Cold Spring Harbor University Press, NY, USA) which is active around neutral pH and belongs to a family of proteases known to the person skilled in the art as subtilisins. A subtilisin is a serine protease produced by Gram-positive bacteria or fungi.

In the next steps of the sample preparation which follow on the lysis step, the target component is further enriched. If the target component is a nucleic acid, the target nucleic acid is normally extracted from the complex lysis mixtures before it is used in a probe-based assay.

There are several methods for the extraction of nucleic acids:
- sequence-dependent or biospecific methods as e.g.:
   - affinity chromatography
   - hybridisation to immobilised probes
- sequence-independent or physico-chemical methods as e.g.:
   - liquid-liquid extraction with e.g. phenol-chloroform
   - precipitation with e.g. pure ethanol
   - extraction with filter paper
   - extraction with micelle-forming agents as cetyl-trimethyl-ammonium-bromide
   - binding to immobilised, intercalating dyes, e.g. acridine derivatives
   - adsorption to silica gel or diatomic earths
   - adsorption to magnetic glass particles (MGP) or organo silane particles under chaotropic conditions.

Particularly interesting for extraction purposes is the adsorption of nucleic acids to a glass surface although other surfaces are possible. Many procedures for isolating nucleic acids from their natural environment have been proposed in recent years by the use of their binding behavior to glass or silica surfaces.

For example, EP 0 389 063 discloses the binding of nucleic acids to silica surface in the presence of chaotropic agents. WO 96/41811 and WO 01/37291 disclose the binding of nucleic acids to the silica surface of magnetic glass particles. Commercial systems that are sold on the market are e.g. the High Pure system and the MagNA Pure system available from Roche Diagnostics, Mannheim, Germany.

In order to enhance or influence the lysis of the biological sample and/ or the binding behaviour of nucleic acids to silica surfaces various agents were used in the prior art.

WO 95/01359 discloses the use of 1- 50 % of different alcohols, polyethylene glycol or trichloro acetic acid in combination with high salt concentrations for binding a nucleic acid to a silica surface.

WO 97/05248 discloses solutions for the isolation and extraction of DNA, RNA and proteins from a biological sample. The solutions include a chaotropic agent, a reducing agent and an organic solvent such as an alcohol.

WO 01/37291 discloses a lysis buffer comprising 50 mM Tris pH 7.0, 15 % (V/V) polidocanol, 5 M guanidinium isothiocyanate and 1 mM dithiothreitol (DTT).

WO 2005/064010 relates to a composition which is used to bind nucleic acids, in an aqueous solution to a solid phase. Said composition contains a guanidinium salt, a buffer substance and a detergent such as Triton-X-100, NP-40, polidocanol and Tween 20. The detergent concentration can be between 5 to 30 %, preferably between 10 to 20 %.

WO 00/09746 and WO 01/60517 describe a vessel with a solution that comprises a guanidinium-salt, a buffer substance, a reducing agent and a detergent. The detergent concentration can be between 5 to 30 %.

WO 2005/012523 and WO 2005/034967 describe compositions which comprise a chaotropic agent, a buffering substance and a detergent in a range of 0.5 to 4.5% (V/V), wherein said detergent is or may be polidocanol.

### Summary of the Invention

Therefore, it was an object of the present invention to provide a new composition for the lysis of a biological sample and/ or for influencing or enhancing the binding behaviour of nucleic acids to silica surfaces.

This problem was solved by the findings of the present invention which is related to a composition comprising a chaotropic agent, a buffering substance, a protease, and 0.5 to 4.9 % (V/V) polidocanol or a derivative thereof.

In another embodiment of the invention, the composition according to the invention is used for the purification of a nucleic acid, for binding a nucleic acid to a solid surface or for the detection of a nucleic acid.

In yet another embodiment of the invention, a method for the detection of a nucleic acid in a biological sample is provided comprising the steps of
a) incubating the biological sample in the presence of a chaotropic agent, a buffering substance, a protease, and 0.5 to 4.9 % (V/V) polidocanol or a derivative thereof,
b) optionally isolating the nucleic acid,
c) optionally amplifying the nucleic acid, and
d) detecting the nucleic acid.

In still another embodiment of the invention, a method for the purification of a nucleic acid in a biological sample is provided comprising the steps of
a) incubating the biological sample in the presence of a chaotropic agent, a buffering substance, a protease, and 0.5 to 4.9 % (V/V) polidocanol or a derivative thereof,
b) isolating the nucleic acid thereby purifying the nucleic acid.

The term "polidocanol" or "polydocanol" relates to a chemical compound or composition consisting of a mixture of polyethylene glycol monododecyl ethers averaging about 9 ethylene oxide groups per molecule. It could be described by the molecular formula (C₂H₄O)ₙC₁₂H₂₆O or HO(CH₂CH₂O)ₙ(CH₂)₁₁CH₃ whereby n is about 9, i.e. the median number of ethylene glycol moieties is about 9 as a result of the production method wherein lauryl alcohol is reacted with ethylene oxide (ethoxylation). This means that the molecular weight is about 600 g/ mol. Other names for this compound are 3,6,9,12,15,18,21,24,27-nonaoxanonatriacontan-1-ol, dodecyl nonaethylene glycol ether, dodecylnonaglycol, polyoxyethylene 9 lauryl ether or laureth 9. The compound is also a suitable emulsifying and solubilizing agent for oil/ water (O/W) types of cosmetic and dermatological emulsions, a topical anaesthetics, a spermicide and surfactant or a sclerosing agent in treatment of varicose veins. The compound can be obtained e.g. from Kolb, Hedingen, Switzerland (Sympatens-AL/090 P). According to the invention, the term "polidocanol" or "polydocanol" shall however also refer to the chemically defined compounds with the formula (C₂H₄O)₉C₁₂H₂₆O or HO(CH₂CH₂O)₉(CH₂)₁₁CH₃. "Polidocanol" is a white ointment-like substance at room temperature and becomes a clear, colorless to slightly yellow liquid at approximately 30°C. Therefore, for preparing polidocanol containing compositions polidocanol can be pipetted when heated e.g. in a water bath to e.g. 37°C or 40°C or added as a solid substance at room temperature. Therefore, according to the invention 2,371 (liquid) polidocanol (at little more than 30° C, preferably 37° C) equal 2.365 kg solid polidocanol (at little less than 30°C, preferably room temperature, i.e. between 20 to 25°C). The resulting compositions or solutions denote the polidocanol contend as % (V/V) or % (W/V). The term "(V/V)" shall mean volume per volume and "(W/V)" shall mean weight per volume. The preferred solvent or major component of the composition shall be water, i.e. preferably these are aqueous compositions or aqueous solutions.

The term "derivative of polidocanol" or "derivative of polidocanol" relates to "polidocanol" or "polydocanol" that is chemically derivatized but has properties, in particular properties in the methods according to the invention, that are identical or very similar to the properties of "polidocanol" or "polydocanol".

Another word for "composition" is solution in the context of the invention.

"Biological samples" are samples which are taken from a plant or an animal (including a human being) and are solid or liquid. Specific examples are described in more detail below.

### Detailed description of the Invention

In an embodiment of the invention, a composition is provided comprising
- a chaotropic agent,
- a buffering substance,
- a protease, and
- 0.5 to 4.9 % (V/V) polidocanol or a derivative thereof.

In a preferred embodiment of the invention, the composition according to the invention comprises 0.5 to 4.5 % (V/V) polidocanol 0.5 to 3 % (V/V) polidocanol or a derivative thereof, preferably the composition comprises 0.75 to 1.75 % (V/V) polidocanol or a derivative thereof.

In another preferred embodiment of the invention, the composition according to the invention comprises 1 to 4.5 % (V/V) polidocanol or a derivative thereof, preferably the composition comprises 1.5 to 3 % (V/V) polidocanol or a derivative thereof.

In another preferred embodiment of the invention, in the composition according to the invention, the chaotropic agent is guanidinium thiocyanate, guanidinium isothiocyanate, guanidinium chloride or urea. However, potassium chlorate (KClO₄) or potassium iodide (KI) is also possible.

In another preferred embodiment of the invention, in the composition according to the invention, the buffering substance is Tris-(hydroxymethyl)-aminomethane (TRIS), phosphate, N-(2-hydroxyethyl)-piperazine-N'-(2-ethanesulfonic acid) (HEPES), acetate or citrate.

In another preferred embodiment of the invention, in the composition according to the invention, the pH of the composition is acidic, preferably the pH of the composition is between 3 and 5.

In still another preferred embodiment of the invention, the composition further comprises a reducing agent, preferably dithiothreitol (DTT).

In a very preferred embodiment of the invention, the composition comprises 4 M guanidinium thiocyanate, 50 mM Na-Citrate, 1% (W/V) DTT, 3% (V/V) polidocanol, pH 4.

In a preferred embodiment of the invention, the composition according to the invention is used for the purification of a nucleic acid, for binding a nucleic acid to a solid surface or for the detection of a nucleic acid.

The invention further contemplates a kit of parts characterized in that it contains the composition according to the invention. Such kits known in the art further comprise plastics ware which can be used during the sample preparation procedure as e.g. microtitre plates in the 96 or 384 well format or just ordinary reaction tubes manufactured e.g. by Eppendorf, Hamburg, Germany and all other reagents for carrying out the method according to the invention. Therefore, the kit can additionally contain a material with an affinity to nucleic acids, preferably the material with an affinity to nucleic acids comprises a material with a silica surface. Preferably, the material with a silica surface is a glass. Most preferably, the material with an affinity to nucleic acids is a composition comprising magnetic glass particles. These components of the kit according to the invention may be provided separately in tubes or storage containers. Depending on the nature of the components, these may be even provided in a single tube or storage container. The kit may further or additionally comprise a washing solution which is suitable for the washing step of the magnetic glass particles when DNA or RNA is bound thereto. This washing solution may contain ethanol and/ or chaotropic agents in a buffered solution or solutions with an acidic pH without ethanol and/ or chaotropic agents as described above. Often the washing solution or other solutions are provided as stock solutions which have to be diluted before use. The kit may further or additionally comprise an eluent or elution buffer, i.e. a solution or a buffer (e.g. 10 mM Tris, 1 mM EDTA, pH 8.0) or pure water to elute the DNA or RNA bound to the magnetic glass particles. Further, additional reagents or buffered solutions may be present which can be used for the purification process of a nucleic acid, i.e. DNA or RNA. Therefore, in an embodiment of the invention, a kit comprising a composition according to the invention is provided. Preferably, the kit additionally contains a material with an affinity to nucleic acids, preferably a material with a silica surface. More preferably, the material with an affinity to nucleic acids is a composition comprising magnetic glass particles. Preferably the kit according to the invention additionally comprises a washing buffer and an elution buffer.

Is preferred embodiments of the invention the kit according to the invention is used for the purification of nucleic acids in research, bioanalytics or diagnostics. In preferred embodiments according to the invention the kit according to the invention or the method according to the invention is use in a high-throughput format, i.e. in an automatized method which allows the analysis of a high number of different samples.

In an embodiment of the invention, a method for the detection of a nucleic acid in a biological sample is provided comprising the steps of
a) incubating the biological sample in the presence of a chaotropic agent, a buffering substance, a protease, and 0.5 to 4.9 % (V/V) polidocanol or a derivative thereof,
b) Optionally isolating the nucleic acid,
c) optionally amplifying the nucleic acid, and
d) detecting the nucleic acid.

In step a) of the method according to the invention, the biological sample is lysed releasing the biological substances including nucleic acids contained in the biological sample. With regard to the general parameters for a lysis procedure to obtain nucleic acids, special reference is made to Sambrook, J., et aL: Molecular Cloning, A Laboratory Manual, 2nd edition (1989) Cold Spring Harbour Laboratory Press, NY, USA, and Ausubel, F., et al.: Current Protocols in Molecular Biology (1987) John Wiley & Sons, Inc., NY, USA. A combination of procedures for lysis using the composition according to the invention is applicable as well. For instance, lysis can be performed using ultrasound, high pressure, by shear forces. It is also often the case, that proteases that degrade the proteins present in the biological sample are added. The protease according to the present invention maybe added in solid form e.g. as a tablet or a powder or in a dissolved form in a buffered or unbuffered solution. Examples for proteases are proteinase K or another protease from Bacillus subtilis described in EP 1 201 753.

The nucleic acid is preferably amplified with the polymerase chain reaction which specifically amplifies target sequences to detectable amounts. Therefore, in a preferred embodiment of the invention, in the amplifying step c) of the method according to the invention, the nucleic acid is amplified by the polymerase chain reaction. Other possible amplification reactions are the ligase Chain Reaction (LCR, Wu, D.Y., and Wallace, R.B., Genomics 4 (1989) 560-569 and Barany, F., Proc. Natl. Acad. Sci. USA 88 (1991) 189-193); Polymerise Ligase Chain Reaction (Barany, F., PCR Methods and Appl. 1 (1991) 5-16); Gap-LCR (PCT Patent Publication No. WO 90/01069); Repair Chain Reaction (EP 0 439 182), 3SR (Kwoh, D.Y., et al., Proc. Natl. Acad. Sci. USA 86 (1989) 1173-1177; Guatelli, C.J., et al., Proc. NatL Acad. Sci. USA 87 (1990) 1874-1878; PCT Patent Publication No. WO 92/08808), and NASBA (US 5,130,238). Further, there are strand displacement amplification (SDA), transciption mediated amplification (TMA), and Qβ-amplification (for a review see e.g. Whelen, A.C., and Persing, D.H., Annu. Rev. Microbiol. 50 (1996) 349-373; Abramson, RD., and Myers, T.W., Current Opinion in Biotechnology 4 (1993) 41-47.

A particularly preferred detection method is the method performed in the TaqMan^{®} instrument disclosed in WO 92/02638 and the corresponding US patents US 5,210,015, US 5,804,375, US 5,487,972. This method exploits the exonuclease activity of a polymerase to generate a signal. In detail, the target nucleic acid component is detected by a process comprising contacting the sample with an oligonucleotide containing a sequence complementary to a region of the target nucleic acid component and a labeled oligonucleotide containing a sequence complementary to a second region of the same target nucleic acid component sequence strand, but not including the nucleic acid sequence defined by the first oligonucleotide, to create a mixture of duplexes during hybridization conditions, wherein the duplexes comprise the target nucleic acid annealed to the first oligonucleotide and to the labeled oligonucleotide such that the 3'-end of the first oligonucleotide is adjacent to the 5'-end of the labeled oligonucleotide. Then this mixture is treated with a template-dependent nucleic acid polymerase having a 5' to 3' nuclease activity under conditions sufficient to permit the 5' to 3' nuclease activity of the polymerase to cleave the annealed, labeled oligonucleotide and release labeled fragments. The signal generated by the hydrolysis of the labeled oligonucleotide is detected and/ or measured. The method performed in the TaqMan® instrument eliminates the need for a solid phase bound reaction complex to be formed and made detectable. In more general terms, a procedure for the purification of a (at least one) target nucleic acid component followed by a detection step is disclosed wherein the amplification and/ or detection reaction is a homogeneous solution-phase.

The nucleic acid may be determined or detected by standard analytical methods known to the person skilled in the art and described e.g. in Sambrook, J., et al.: Molecular Cloning (1989) Cold Spring Harbor University Press, NY, USA or in Lottspeich, F., and Zorbas, H. (eds.), Bioanalytik, 1st edition (1998) Spektrum Akademischer Verlag GmbH, Heidelberg, Berlin, Germany. Preferably, the amount of the nucleic acid is determined with the methods described therein. There may be also further purification steps before the DNA detection step is carried out as e.g. a precipitation step. The detection methods may include but are not limited to the binding or intercalating of specific dyes as ethidiumbromide which intercalates into the double-stranded DNA and changes its fluorescence thereafter. The purified DNA may also be separated by electrophoretic methods optionally after a restriction digest and visualized thereafter. There are also probe-based assays which exploit the oligonucleotide hybridisation to specific sequences and subsequent detection of the hybrid, It is also possible to sequence the DNA after further steps known to the expert in the field. Other methods apply a diversity of DNA sequences to a silicon chip to which specific probes are bound and yield a signal when a complementary sequences bind.

In an embodiment of the invention, a method for the purification of a nucleic acid in a biological sample is provided comprising the steps of
a) incubating the biological sample in the presence of a chaotropic agent, a buffering substance, a protease, and 0.5 to 4.9 % (V/V) polidocanol or a derivative thereof,
b) isolating the nucleic acid thereby purifying the nucleic acid.

The incubation conditions of step a) of both methods according to the invention are preferably prepared by adding a composition according to the invention to the biological sample. In a preferred embodiment of the invention, in step a) of the method according to the invention, the biological sample is incubated in the presence of a chaotropic agent, a buffering substance, a protease, and 0.5 to 4.5 % (V/V) polidocanol or a derivative thereof, 0.5 to 3 % (V/V) polidocanol or a derivative thereof, preferably 0.75 to 1.75 % (V/V) polidocanol or a derivative thereof.

Preferably, the isolating step b) of the method according to the invention comprises binding the nucleic acid to a material with an affinity to nucleic acids, preferably a material with a silica surface, optionally washing the nucleic acid bound to the material and eluting the nucleic acid from the material. Preferably, the material with a silica surface is a composition comprising magnetic glass particles. In the most preferred embodiment, the washing step is not optional.

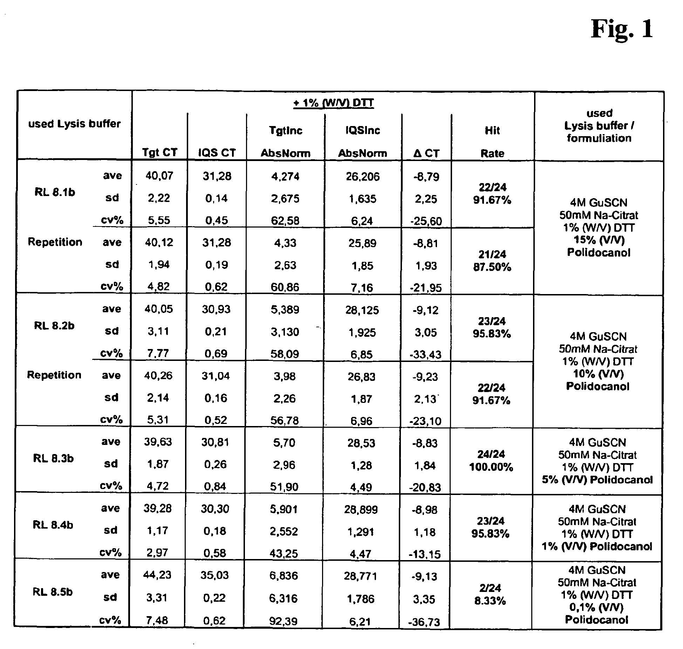

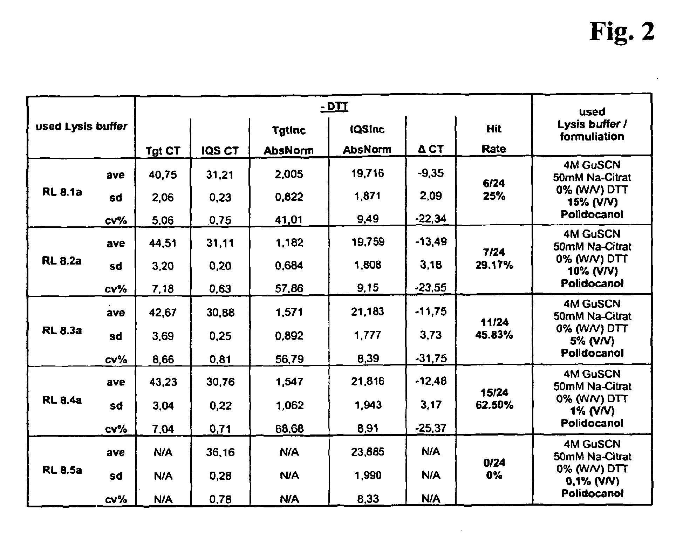

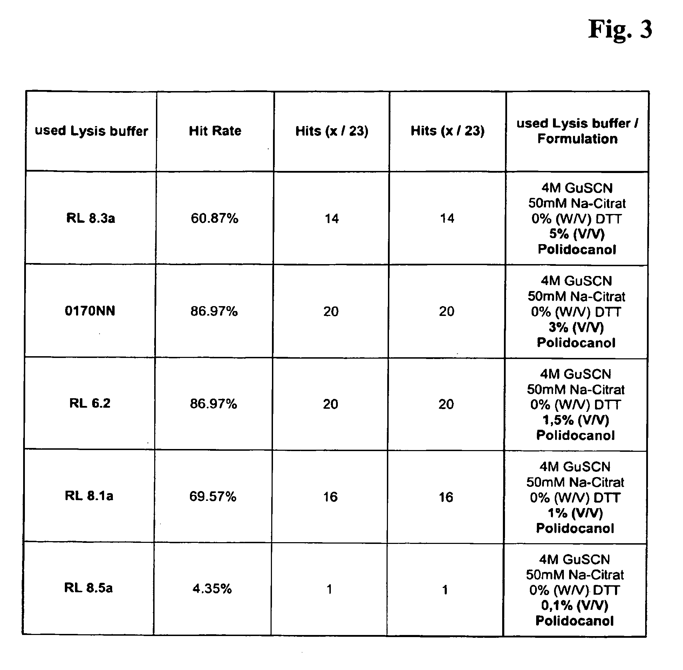

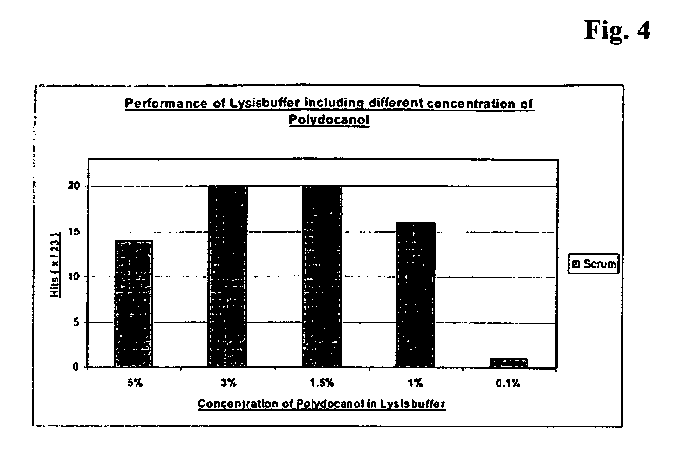

## Claims

1. A composition comprising
- a chaotropic agent,
- a buffering substance,
- a protease, and
- 0.5 to 4.9 % (V/V) polidocanol or a derivative thereof.

2. The composition according to claim 1 wherein the composition comprises 0.5 to 3 % (V/V) polidocanol or a derivative thereof, preferably the composition comprises 0.75 to 1.75 % (V/V) polidocanol or a derivative thereof.

3. The composition according to claim 1 wherein the composition comprises 1 to 4.5 % (V/V) polidocanol or a derivative thereof, preferably the composition comprises 1.5 to 3 % (V/V) polidocanol or a derivative thereof.

4. The composition according to any of the claims 1 to 3 wherein the chaotropic agent is guanidinium thiocyanate, guanidinium chloride or urea.

5. The composition according to any of the claims 1 to 4 wherein the buffering substance is Tris-(hydroxymethyl)-aminomethane (TRIS), phosphate, N-(2-hydroxyethyl)-piperazine-N'-(2-ethanesulfonic acid) (HEPES), acetate or citrate.

6. The composition according to any of the claims 1 to 5 wherein the pH of the composition is acidic, preferably the pH of the composition is between 3 and 5.

7. The composition according to any of the claims 1 to 6 wherein the composition further comprises a reducing agent.

8. The composition according to any of the claims 1 to 7 wherein the composition comprises 4 M guanidinium thiocyanate, 50 mM Na-Citrate, 1 % (W/V) DTT, 3 % (V/V) polidocanol, pH 4.

9. Use of a composition according to any of the claims 1 to 8 for the purification of a nucleic acid, for binding a nucleic acid to a solid surface or for the detection of a nucleic acid.

10. A kit comprising a composition according to any of the claims 1 to 8.

11. The kit according to claim 10 **characterized in that** the kit additionally contains a material with an affinity to nucleic acids, preferably a material with a silica surface.

12. The kit according to claim 11 **characterized in that** the material with an affinity to nucleic acids is a composition comprising magnetic glass particles.

13. The kit according to any of the claims 10 to 12 **characterized in that** the kit additionally comprises a washing buffer and an elution buffer.

14. A method for the detection of a nucleic acid in a biological sample comprising the steps of
a) incubating the biological sample in the presence of a chaotropic agent, a buffering substance, a protease and 0.5 to 4.9 % (V/V) polidocanol or a derivative thereof,
b) optionally isolating the nucleic acid,
c) optionally amplifying the nucleic acid, and
d) detecting the nucleic acid.

15. The method according to claim 14 whereby in the amplifying step c) the nucleic acid is amplified by the polymerase chain reaction.

16. A method for the purification of a nucleic acid in a biological sample comprising the steps of
a) incubating the biological sample in the presence of a chaotropic agent, a buffering substance, a protease and 0.5 to 4.9 % (V/V) polidocanol or a derivative thereof,
b) isolating the nucleic acid thereby purifying the nucleic acid.

17. The method according to any of the claims 14 to 16 wherein in step a) the biological sample is incubated in the presence of a chaotropic agent, a buffering substance, and 0.5 to 3 % (V/V) polidocanol or a derivative thereof, preferably 0.75 to 1.75 % (V/V) polidocanol or a derivative thereof.

18. The method according to any of the claims 14 to 17 whereby the isolating step b) comprises binding the nucleic acid to a material with an affinity to nucleic acids, preferably a material with a silica surface, optionally washing the nucleic acid bound to the material and eluting the nucleic acid from the material.

19. The method according to claim 18 **characterized in that** the material with a silica surface is a composition comprising magnetic glass particles.

20. The method according to any of the claims 14 to 19 **characterized in that** the biological sample is a fluid from the human or animal body, preferably blood, blood plasma, blood serum or urine.

21. The method according to any of the claims 14 to 20 **characterized in that** the nucleic acid comprises DNA or RNA or both.

22. The method according to claim 21 **characterized in that** the DNA or RNA or both is derived from a microorganism or a virus, preferably the hepatitis A virus, hepatitis B virus, the hepatitis C virus, the human immunodeficiency virus or the cytomegalovirus.

## Patentansprüche

1. Zusammensetzung, umfassend
- ein chaotropes Mittel,
- eine Puffersubstanz,
- eine Protease und
- 0,5 bis 4,9 Vol.-% Polidocanol oder ein Derivat davon.

2. Zusammensetzung nach Anspruch 1, wobei die Zusammensetzung 0,5 bis 3 Vol.-% Polidocanol oder ein Derivat davon, vorzugsweise 0,75 bis 1,75 Vol.-% Polidocanol oder ein Derivat davon umfasst.

3. Zusammensetzung nach Anspruch 1, wobei die Zusammensetzung 1 bis 4,5 Vol.-% Polidocanol oder ein Derivat davon, vorzugsweise 1,5 bis 3 Vol.-% Polidocanol oder ein Derivat davon umfasst.

4. Zusammensetzung nach einem der Ansprüche 1 bis 3, wobei es sich bei dem chaotropen Mittel um Guanidiniumthiocyanat, Guanidiniumchlorid oder Harnstoff handelt.

5. Zusammensetzung nach einem der Ansprüche 1 bis 4, wobei es sich bei der Puffersubstanz um Tris-(hydroxymethyl)-aminomethan (TRIS), Phosphat, N-(2-Hydroxyethyl)-piperazin-N'-(2-ethansulfonsäure) (HEPES), Acetat oder Citrat handelt.

6. Zusammensetzung nach einem der Ansprüche 1 bis 5, wobei der pH-Wert der Zusammensetzung im sauren Bereich, vorzugsweise zwischen 3 und 5 liegt.

7. Zusammensetzung nach einem der Ansprüche 1 bis 6, wobei die Zusammensetzung ferner ein Reduktionsmittel aufweist.

8. Zusammensetzung nach einem der Ansprüche 1 bis 7, wobei die Zusammensetzung 4 M Guanidiniumthiocyanat, 50 mM Natriumcitrat, 1% (w/v) DTT, 3 Vol.-% Polidocanol, pH 4, umfasst.

9. Verwendung einer Zusammensetzung nach einem der Ansprüche 1 bis 8 zur Aufreinigung einer Nukleinsäure für die Bindung einer Nukleinsäure an eine feste Oberfläche oder für den Nachweis einer Nukleinsäure.

10. Kit, umfassend eine Zusammensetzung nach einem der Ansprüche 1 bis 8.

11. Kit nach Anspruch 10, **dadurch gekennzeichnet, dass** das Kit zusätzlich ein Material mit einer Affinität zu Nukleinsäuren, vorzugsweise ein Material mit einer Siliciumdioxid-Oberfläche enthält.

12. Kit nach Anspruch 11, **dadurch gekennzeichnet, dass** es sich bei dem Material mit einer Affinität zu Nukleinsäuren um eine magnetische Glaspartikel umfassende Zusammensetzung handelt.

13. Kit nach einem der Ansprüche 10 bis 12, **dadurch gekennzeichnet, dass** das Kit zusätzlich einen Waschpuffer und einen Elutionspuffer umfasst.

14. Verfahren zum Nachweis einer Nukleinsäure in einer biologischen Probe, das die folgenden Schritte umfasst:
a) Inkubieren der biologischen Probe in Gegenwart eines chaotropen Mittels, einer Puffersubstanz, einer Protease sowie von 0,5 bis 4,9 Vol.-% Polidocanol oder eines Derivats davon,
b) gegebenenfalls Isolieren der Nukleinsäure,
c) gegebenenfalls Amplifizieren der Nukleinsäure und
d) Nachweisen der Nukleinsäure.

15. Verfahren nach Anspruch 14, wobei im Amplifizierungsschritt c) die Nukleinsäure mit der Polymerasekettenreaktion amplifiziert wird.

16. Verfahren zur Aufreinigung einer Nukleinsäure in einer biologischen Probe, das die folgenden Schritte umfasst:
a) Inkubieren der biologischen Probe in Gegenwart eines chaotropen Mittels, einer Puffersubstanz, einer Protease sowie von 0,5 bis 4,9 Vol.-% Polidocanol oder eines Derivats davon,
b) Isolieren der Nukleinsäure, wodurch die Nukleinsäure aufgereinigt wird.

17. Verfahren nach einem der Ansprüche 14 bis 16, wobei man in Schritt a) die biologische Probe in Gegenwart eines chaotropen Mittels, einer Puffersubstanz sowie von 0,5 bis 3 Vol.-% Polidocanol oder eines Derivats davon, vorzugsweise von 0,75 bis 1,75 von.-% Polidocanol oder eines Derivats davon inkubiert.

18. Verfahren nach einem der Ansprüche 14 bis 17, wobei man im Isolierungsschritt b) die Nukleinsäure an ein Material mit einer Affinität zu Nukleinsäuren, vorzugsweise ein Material mit einer Siliciumdioxid-Oberfläche bindet, gegebenenfalls die an dem Material gebundene Nukleinsäure wäscht und die Nukleinsäure von dem Material eluiert.

19. Verfahren nach Anspruch 18, **dadurch gekennzeichnet, dass** es sich bei dem Material mit einer Siliciumdioxid-Oberfläche um eine magnetische Glaspartikel umfassende Zusammensetzung handelt.

20. Verfahren nach einem der Ansprüche 14 bis 19, **dadurch gekennzeichnet, dass** es sich bei der biologischen Probe um eine Flüssigkeit aus dem menschlichen oder tierischen Körper handelt, vorzugsweise Blut, Blutplasma, Blutserum oder Urin.

21. Verfahren nach einem der Ansprüche 14 bis 20, **dadurch gekennzeichnet, dass** die Nukleinsäure DNA oder RNA oder sowohl DNA als auch RNA umfasst.

22. Verfahren nach Anspruch 21, **dadurch gekennzeichnet, dass** die DNA oder RNA bzw. sowohl die DNA als auch die RNA aus einem Mikroorganismus oder einem Virus, vorzugsweise dem Hepatitis-A-Virus, Hepatitis-B-Virus, dem Hepatitis-C-Virus, dem menschlichen Immunschwächevirus oder dem Cytomegalievirus, stammt.

## Revendications

1. Composition comprenant
- un agent chaotropique,
- une substance tampon,
- une protéase, et
- 0,5 à 4,9 % (v/v) de polidocanol ou d'un dérivé de celui-ci.

2. Composition selon la revendication 1, dans laquelle la composition comprend 0,5 à 3 % (v/v) de polidocanol ou d'un dérivé de celui-ci, de préférence la composition comprend de 0,75 à 1,75 % (v/v) de polidocanol ou d'un dérivé de celui-ci.

3. Composition selon la revendication 1, dans laquelle la composition comprend 1 à 4,5 % (v/v) de polidocanol ou d'un dérivé de celui-ci, de préférence la composition comprend 1,5 à 3 % (v/v) de polidocanol ou d'un dérivé de celui-ci.

4. Composition selon l'une quelconque des revendications 1 à 3, dans laquelle l'agent chaotropique est le thiocyanate de guanidinium, le chlorure de guanidinium ou l'urée.

5. Composition selon l'une quelconque des revendications 1 à 4, dans laquelle la substance tampon est le Tris-(hydroxyméthyl)-aminométhane (TRIS), le phosphate, l'acide N-(2-hydroxyéthyl)-pipérazine-N'-(2-éthane sulfonique) (HEPES), l'acétate ou le citrate.

6. Composition selon l'une quelconque des revendications 1 à 5, dans laquelle le pH de la composition est acide, de préférence le pH de la composition est compris entre 3 et 5.

7. Composition selon l'une quelconque des revendications 1 à 6, dans laquelle la composition comprend en outre un agent réducteur.

8. Composition selon l'une quelconque des revendications 1 à 7, dans laquelle la composition comprend 4 M de thiocyanate de guanidinium, 50 mM de citrate de sodium, 1 % (p/v) de DTT, 3 % (v/v) de polidocanol, pH4.

9. Utilisation d'une composition selon l'une quelconque des revendications 1 à 8 pour la purification d'un acide nucléique, pour la liaison d'un acide nucléique à une surface solide ou pour la détection d'un acide nucléique.

10. Trousse comprenant une composition selon l'une quelconque des revendications 1 à 8.

11. Trousse selon la revendication 10, **caractérisée en ce que** la trousse contient en outre un matériau ayant une affinité pour les acides nucléiques, de préférence un matériau avec une surface de silice.

12. Trousse selon la revendication 11, **caractérisée en ce que** le matériau ayant une affinité pour les acides nucléiques est une composition comprenant des particules de verre magnétiques.

13. Trousse selon l'une quelconque des revendications 10 à 12, **caractérisée en ce que** la trousse comprend en outre un tampon de lavage et un tampon d'élution.

14. Procédé pour la détection d'un acide nucléique dans un échantillon biologique, comprenant les étapes de :
a) incubation de l'échantillon biologique en présence d'un agent chaotropique, d'une substance tampon, d'une protéase et de 0,5 à 4,9 % (v/v) de polidocanol ou d'un dérivé de celui-ci,
b) éventuellement isolement de l'acide nucléique,
c) éventuellement amplification de l'acide nucléique, et
d) détection de l'acide nucléique.

15. Procédé selon la revendication 14 par lequel, dans l'étape d'amplification c) l'acide nucléique est amplifié par la réaction en chaîne par polymérase.

16. Procédé pour la purification d'un acide nucléique dans un échantillon biologique, comprenant les étapes de
a) incubation de l'échantillon biologique en présence d'un agent chaotropique, d'une substance tampon, d'une protéase et de 0,5 à 4,9 % (v/v) de polidocanol ou d'un dérivé de celui-ci,
b) l'isolement de l'acide nucléique purifiant ainsi l'acide nucléique.

17. Procédé selon l'une quelconque des revendications 14 à 16, dans lequel à l'étape a) l'échantillon biologique est incubé en présence d'un agent chaotropique, d'une substance tampon, et de 0,5 à 3 % (v/v) de polidocanol ou d'un dérivé de celui-ci, de préférence de 0,75 à 1,75 % (v/v) de polidocanol ou d'un dérivé de celui-ci.

18. Procédé selon l'une quelconque des revendications 14 à 17 par lequel l'étape d'isolement b) comprend la liaison de l'acide nucléique à un matériau ayant une affinité pour les acides nucléiques, de préférence un matériau avec une surface de silice, éventuellement le lavage de l'acide nucléique lié au matériau et l'élution de l'acide nucléique du matériau.

19. Procédé selon la revendication 18, **caractérisé en ce que** le matériau avec une surface de silice est une composition comprenant des particules de verre magnétiques.

20. Procédé selon l'une quelconque des revendications 14 à 19, **caractérisé en ce que** l'échantillon biologique est un fluide provenant du corps humain ou animal, de préférence le sang, le plasma sanguin, le sérum sanguin ou l'urine.

21. Procédé selon l'une quelconque des revendications 14 à 20, **caractérisé en ce que** l'acide nucléique comprend de l'ADN ou de l'ARN ou les deux.

22. Procédé selon la revendication 21, **caractérisé en ce que** l'ADN ou l'ARN ou les deux est(sont) dérivé(s) d'un micro-organisme ou d'un virus, de préférence le virus de l'hépatite A, le virus de l'hépatite B, le virus de l'hépatite C, le virus de l'immunodéficience humaine ou le cytomégalovirus.
